# EUROPEAN PATENT SPECIFICATION

(11) **EP 4 206 194 B1**
(45) Date of publication and mention of the grant of the patent: **11.11.2026**
(21) Application number: 21860544.2
(22) Date of filing: 30.08.2021
(51) Int. Cl.: C07D 403/14, A61P 29/00, A61K 31/506, A61K 31/416

(54) **SALT OF RHO-ASSOCIATED PROTEIN KINASE INHIBITOR, SOLID FORM OF THE SALT, PREPARATION METHOD FOR SAME, AND USES THEREOF**
SALZ EINES RHO-ASSOZIIERTEN PROTEINKINASEHEMMERS, FESTE FORM DES SALZES, HERSTELLUNGSVERFAHREN DAFÜR UND VERWENDUNGEN DAVON
SEL D'INHIBITEUR DE PROTÉINE KINASE ASSOCIÉE À RHO, FORME SOLIDE DU SEL, SON PROCÉDÉ DE PRÉPARATION ET SES UTILISATIONS

(30) Priority: 31.08.2020 WO PCT/CN2020/112500
(43) Date of publication of application: 05.07.2023
(73) Proprietor: Beijing Tide Pharmaceutical Co., Ltd., Beijing 100176 (CN)
(72) Inventor: ZHAO, Yanping, Beijing 100176 (CN); WANG, Hongjun, Beijing 100176 (CN); FENG, Zewang, Beijing 100176 (CN); TIAN, Nana, Beijing 100176 (CN); WEI, Lai, Beijing 100176 (CN); CAO, Xiangrong, Beijing 100176 (CN); CHEN, Jie, Beijing 100176 (CN)
(74) Representative: Turner, Craig Robert
(86) International application number: PCT/CN2021/115198
(87) International publication number: WO 2022/042712

(56) References cited:
- WO-A1-2019/001572
- WO-A1-2019/001572
- WO-A1-2020/177587
- WO-A1-2020/177587
- WO-A1-2020/259528
- WO-A1-2020/259528
- WO-A1-2023/041026
- HIFIKER, R.: "Polymorphism in the Pharmaceutical Industry", January 2006, WILEY-VCH, Weinheim, ISBN: 978-3-527-31146-0, article HILFIKER R., BLATTER F., VON RAUMER M.: "Relevance of Solid-state Properties for Pharmaceutical Products", pages: 1 - 19, XP002528052

## Description

### FIELD OF THE INVENTION

The present invention relates to crystalline form I of (6-(4-((4-(1H-pyrazol-4-yl)phenyl)atnino)pyrimidin-2-yl)-1-methyl-1H-indol-2-yl)(3,3-difluoroazetidine-1-yl)methanone (hereinafter referred to as "compound A"), a method for preparing the crystalline form I, a pharmaceutical composition comprising crystalline form I, and crystalline form I for use as a Rho-associated protein kinase (ROCK) inhibitor, preferably a selective ROCK2 inhibitor.

### BACKGROUND OF THE INVENTION

Rho-associated protein kinase (ROCK) is a serine/threonine kinase from the AGC kinase family, and comprises two isoforms, ROCK1 and ROCK2. ROCK1 and ROCK2 are expressed and regulated differently in specific tissues. For example, ROCK1 is ubiquitously expressed at a relatively high level, while ROCK2 is preferentially expressed in heart, brain and skeletal muscle. ROCK is the first downstream effector of the Rho protein discovered, and its biological function is achieved by phosphorylating the downstream effector proteins (MLC, Lin-11, Isl-1, LIMK, ERM, MARCKS, CRMP-2, *etc*.). Studies have shown that various diseases (e.g., pulmonary fibrosis, cardiac-cerebral vascular disease, neurological disease and cancer *etc*.) are related to the pathways mediated by ROCK. As such, ROCK is considered as an important target in the development of novel drugs.

WO 2019/001572 A1 discloses a Rho-associated protein kinase inhibitor, a pharmaceutical composition comprising the same, a preparation method thereof, and a use of the same in preventing or treating a disease mediated by Rho-associated protein kinase (ROCK).

WO 2020/259528 A1 discloses a method for preventing, alleviating and/or treating idiopathic pulmonary fibrosis, which comprises administering an effective amount of a Rho-associated protein kinase inhibitor, or a pharmaceutically acceptable salt, ester, stereoisomer, polymorph, solvate, N-oxide, isotope-labeled compound, metabolite or prodrug thereof to an individual in need thereof.

WO 2020/177587 A1 discloses a method for preventing, alleviating and/or treating fatty liver disease and/or steatohepatitis. The method comprises administering, to an individual in need thereof, an effective amount of a Rho-associated protein kinase inhibitor or a pharmaceutically acceptable salt, an ester, a stereoisomer, a polymorph, a solvate, an N-oxide, an isotopically labeled compound, a metabolite or a prodrug thereof.

WO 2023/041026 A1 discloses a solid form of a Rho-associated protein kinase inhibitor (6-(4-((4-(1H-pyrazol-4-yl)phenyl)amino)pyrimidin-2-yl)-1-methyl-1H-indol-2-yl)(3,3-difluoroazetidin-1-yl)methanone or a solvate thereof, a method for preparing the solid form, a pharmaceutical composition comprising the solid form, and a use of the solid form as a Rho-associated protein kinase (ROCK) inhibitor, preferably a selective ROCK2 inhibitor.

Hilfiker R., Blatter F., von Raumer M. et al. ("Relevance of Solid-state Properties for Pharmaceutical Products" In: Hifiker, R.: "Polymorphism in the Pharmaceutical Industry", January 2006 (2006-01), Wiley-VCH, Weinheim, XP002528052, ISBN: 978-3-527-31146-0, pages 1-19) is concerned with the development of salts and solid forms of interesting molecules in the pharmaceutical industry.

The applicant has discovered that (6-(4-((4-(1H-pyrazol-4-yl)phenyl)amino)pyrimidin-2-yl)-1-methyl-1H-indol-2-yl)(3,3-difluoroazetidin-1-yl)methanone can be used as a potent Rho-associated protein kinase (ROCK) inhibitor (see PCT/CN2018/093713), but salts of the compound and solid forms thereof have not yet been reported.

### SUMMARY OF THE INVENTION

The invention claimed is as set out in appended claims 1 to 11. Disclosures herein which are outside the scope of the appended claims are not part of the invention claimed. In one aspect, the present disclosure provides a salt of compound A ((6-(4-((4-(1H-pyrazol-4-yl)phenyl)atnino)pyrimidin-2-yl)-1-methyl-1H-indol-2-yl)(3,3-difluoroazetidin-1-yl)methanone) as shown below:

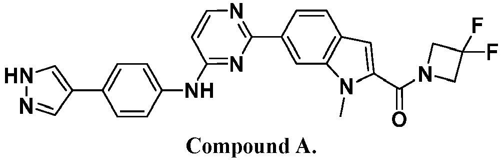

In another aspect, the present disclosure provides crystalline forms of the compound A salts and solvates thereof.

The preferred crystalline forms of the present disclosure not only have an excellent effect in preventing or treating a disease mediated by the Rho-associated protein kinase (ROCK), but also have other advantages. For example, the preferred crystalline forms of the present disclosure have excellent physical properties (including solubility, dissolution rate, light resistance, low hygroscopicity, high temperature resistance, high humidity resistance, fluidity, and the like), and the preferred crystalline forms of the present disclosure may have superior properties in terms of bioavailability, physical and/or chemical stability, and ease of preparation. The preferred crystalline forms of the present disclosure have good powder properties, are more suitable and convenient for mass production and for forming a formulation, can reduce irritation and enhance absorption, solve problems in metabolic rates, significantly decrease toxicity resulted from drug accumulation, improve safety, and effectively ensure the quality and efficacy of the pharmaceutical products.

In another aspect, the present disclosure provides methods for preparing the crystalline forms of the present disclosure.

In another aspect, the present disclosure provides a pharmaceutical composition comprising any one or more of the crystalline forms of the present disclosure and one or more pharmaceutically acceptable carriers.

In another aspect, the present disclosure provides use of the crystalline forms of the present disclosure in the manufacture of a medicament as a Rho-associated protein kinase (ROCK) inhibitor, preferably selective ROCK2 inhibitor.

### BRIEF DESCRIPTION OF THE DRAWINGS

Figure 1 is an X-ray powder diffraction (XRPD) pattern of crystalline form I of compound A monohydrochloride monohydrate.
Figure 2 is a differential scanning calorimetry (DSC) graph of crystalline form I of compound A monohydrochloride monohydrate.
Figure 3 is a thermogravimetric analysis (TGA) graph of crystalline form I of compound A monohydrochloride monohydrate.
Figure 4 is a scanning electron microscope image of crystalline form I of compound A monohydrochloride monohydrate.
Figure 5 is an XRPD pattern of crystalline form II of compound A monohydrochloride dihydrate.
Figure 6 is a DSC-TGA graph of crystalline form II of compound A monohydrochloride dihydrate.
Figure 7 is a scanning electron microscope image of crystalline form II of compound A monohydrochloride dihydrate.
Figure 8 is an XRPD pattern of crystalline form III of compound A monohydrochloride sesquihydrate.
Figure 9 is a DSC-TGA pattern of crystalline form III of compound A monohydrochloride sesquihydrate.
Figure 10 is a scanning electron microscope image of crystalline form III of compound A monohydrochloride sesquihydrate.
Figure 11 is an XRPD pattern of crystalline form IV of compound A monohydrochloride mono-dimethyl sulfoxide complex.
Figure 12 is a DSC graph of crystalline form IV of compound A monohydrochloride mono-dimethyl sulfoxide complex.
Figure 13 is a TGA graph of crystalline form IV of compound A monohydrochloride mono-dimethyl sulfoxide complex.
Figure 14 is a scanning electron microscope image of crystalline form IV of compound A monohydrochloride mono-dimethyl sulfoxide complex.
Figure 15 is an XRPD pattern of the samples obtained in the solid stability test of Experimental Example 2.
Figure 16 is an XRPD pattern of the samples before and after the DVS test of Experimental Example 4.
Figure 17 is an XRPD pattern of the samples before and after the crystalline form transformation test in water of Experimental Example 5.
Figure 18 is an XRPD pattern of the samples obtained in the grinding test of Experimental Example 6.
Figure 19 is an XRPD spectrum of the samples obtained in the solid stability test of Experimental Example 7.
Figure 20 is a TGA graph of the samples obtained in the solid stability test of Experimental Example 7.

### DETAILED DESCRIPTION OF THE INVENTION

### DEFINITIONS

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that most of the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

The word "about" as used herein refers to, as appreciated by a person skilled in the art, a range within the acceptable standard error of a value, such as ±0.05, ±0.1, ±0.2, ±0.3, ±1, ±2 or ±3, *etc.*

The term "solid form" as used herein includes all solid forms of compound A or any solvate thereof, such as a crystalline form or amorphous form.

The term "amorphous" as used herein refers to any solid substance which lacks order in three dimensions. In some instances, amorphous solids may be characterized by known techniques, including XRPD crystallography, solid state nuclear magnet resonance (ssNMR) spectroscopy, DSC, or some combination of these techniques. As illustrated below, amorphous solids give diffuse XRPD patterns, typically comprised of one or two broad peaks (i.e., peaks having base widths of about 5° 20 or greater).

The term "crystalline form" or "crystal" as used herein refers to any solid substance exhibiting three-dimensional order, which in contrast to an amorphous solid substance, gives a distinctive XRPD pattern with sharply defined peaks.

The term "X-ray powder diffraction pattern (XRPD pattern)" as used herein refers to the experimentally observed diffractogram or parameters derived therefrom. XRPD patterns are usually characterized by peak positions (abscissa) and peak intensities (ordinate).

The term "2θ" as used herein refers to the peak position in degrees based on the experimental setup of the X-ray diffraction experiment and is a common abscissa unit in diffraction patterns. The experimental setup requires that if a reflection is diffracted when the incoming beam forms an angle theta (θ) with a certain lattice plane, the reflected beam is recorded at an angle 2 theta (2θ). It should be understood that reference herein to specific 20 values for a specific solid form is intended to mean the 20 values (in degrees) as measured using the X-ray diffraction experimental conditions as described herein. For example, as described herein, Cu-Kα (Kα1 (Å): 1.540598 and Kα2 (Å): 1.544426 Å) was used as the source of radiation.

As used herein, "I%" refers to the percentage of peak intensity.

The term "differential scanning calorimetry (DSC) graph" as used herein refers to a curve recorded on a differential scanning calorimeter.

The term "thermogravimetric analysis (TGA) graph" as used herein refers to a curve recorded on a thermogravimetric analyzer.

As used herein, the term "essentially the same" with reference to X-ray diffraction peak positions means that typical peak position and intensity variability are taken into account. For example, one skilled in the art will appreciate that the peak positions (2θ) will show some variability, typically as much as 0.1 to 0.2 degree, as well as on the apparatus being used to measure the diffraction. Further, one skilled in the art will appreciate that relative peak intensities will show inter-apparatus variability as well as variability due to degree of crystallinity, preferred orientation, prepared sample surface, and other factors known to those skilled in the art. Similarly, as used herein, "essentially the same" with reference to the DSC graph is intended to also encompass the variabilities associated with these analytical techniques, which are known to those of skill in the art. For example, a differential scanning calorimetry graph will typically have a variability of up to ±0.2°C for well defined peaks, and even larger for broad lines (e.g., up to ±1°C).

The liquid nuclear magnetic resonance spectrum in the present application is preferably collected on a Bruker Advance 300 nuclear magnetic resonance spectrometer, with DMSO-*d₆* as the solvent, unless otherwise stated.

The polarization microscopy data in the present application is preferably collected on Polarizing Microscope ECLIPSE LV100POL (Nikon, JPN).

Numerical ranges (e.g., "1 to 10", "1 to 6", "2 to 10", "2 to 6", "3 to 10", "5 to 10", "3 to 6"), *etc.* as used herein encompass any point within the numerical range (for example, 1, 2, 3, 4, 5, 6, 7, 8, 9, or 10).

The prepared salt or crystalline form thereof may be recovered by methods including decantation, centrifugation, evaporation, gravity filtration, suction filtration, or any other technique for the recovery of solids under pressure or under reduced pressure. The recovered solid may optionally be dried. "Drying" in the present invention is carried out under reduced pressure (preferably in vacuum) until the residual solvent content is lowered within the limits given in the International Conference on Harmonisation of Technical Requirements for Registration of Pharmaceuticals for Human Use ("ICH") guidelines. The residual solvent content depends on the type of the solvent, but does not exceed about 5000 ppm, or preferably about 4000 ppm, or more preferably about 3000 ppm. Drying may be carried out in a tray dryer, vacuum oven, air oven, cone vacuum dryer, rotary vacuum dryer, fluidized bed dryer, spin flash dryer, flash dryer, or the like. The drying may be carried out at temperatures less than about 100°C, less than about 80°C, less than about 60°C, less than about 50°C, less than about 30°C, or any other suitable temperatures, at atmospheric pressure or under a reduced pressure (preferably in vacuum) for any desired period (e.g., about 1, 2, 3, 5, 10, 15, 20, 24 hours or overnight) until the desired result is achieved, as long as the salt is not degraded in quality. The drying can be carried out any desired times until the desired product quality is achieved. The dried product may optionally be subjected to a size reduction procedure to produce desired particle sizes. Milling or micronization may be performed before drying, or after the completion of drying of the product. Techniques that may be used for particle size reduction include, without limitation, ball, roller and hammer milling, and jet milling.

The term "anhydrous" as used herein preferably means a crystalline form in which no water molecule is comprised as a structural element.

### Salt of compound A, crystalline form thereof and preparation method therefor

Disclosed herein is a salt of compound A, 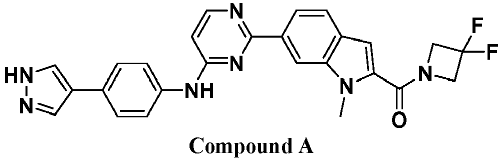 which is an inorganic acid salt or an organic acid salt.

In an embodiment, the present invention provides crystalline form I of compound A monohydrochloride monohydrate, 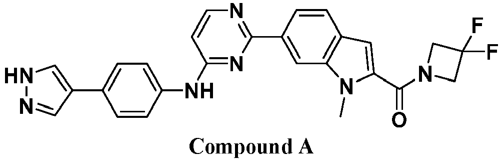 wherein the crystalline form I has an XRPD pattern comprising characteristic peaks at diffraction angles (2θ) of about 8.3±0.2°, 12.6±0.2° and 18.5±0.2°.

In preferred embodiments, the crystalline form I has an XRPD pattern comprising characteristic peaks at diffraction angles (2θ) of about 8.3±0.2°, 12.6±0.2°, 16.5±0.2°, 17.3±0.2°, 17.8±0.2°, 18.5±0.2°, 24.4±0.2°, 26.0±0.2° and 29.1±0.2°.

In more preferred embodiments, the crystalline form I has an XRPD pattern comprising characteristic peaks at diffraction angles (2θ) of about 8.3±0.2°, 11.2±0.2°, 12.6±0.2°, 16.5±0.2°, 17.3±0.2°, 17.8±0.2°, 18.5±0.2°, 19.5±0.2°, 19.8±0.2°, 20.6±0.2°, 21.0±0.2°, 21.7±0.2°, 23.7±0.2°, 24.4±0.2°, 25.5±0.2°, 26.0±0.2°, 29.1±0.2° and 33.6±0.2°.

In more preferred embodiments, the crystalline form I has an XRPD pattern comprising peaks at the following diffraction angles (2θ):

| **Peak No.** | **2θ(°) ± 0.2°** | **I%** | **Peak No.** | **2θ(°) ± 0.2°** | **I%** | **Peak No.** | **2θ(°) ± 0.2°** | **I%** |
|---|---|---|---|---|---|---|---|---|
| 1 | 8.3 | 78.1 | 13 | 21.0 | 15.9 | 25 | 30.0 | 8.2 |
| 2 | 11.2 | 18.7 | 14 | 21.7 | 15.7 | 26 | 31.6 | 4.6 |
| 3 | 12.6 | 100 | 15 | 22.5 | 6.5 | 27 | 32.0 | 4.9 |
| 4 | 13.6 | 8.3 | 16 | 22.8 | 5.9 | 28 | 32.6 | 4.1 |
| 5 | 15.7 | 4.9 | 17 | 23.3 | 9.5 | 29 | 33.6 | 13 |
| 6 | 16.5 | 22.5 | 18 | 23.7 | 15.3 | 30 | 34.4 | 5.9 |
| 7 | 17.3 | 38.6 | 19 | 24.4 | 23.2 | 31 | 34.8 | 6 |
| 8 | 17.8 | 31.5 | 20 | 25.0 | 9.7 | 32 | 35.7 | 7.1 |
| 9 | 18.5 | 65.3 | 21 | 25.5 | 18.2 | 33 | 37.4 | 4.9 |
| 10 | 19.5 | 10.6 | 22 | 26.0 | 64.2 | 34 | 38.4 | 4.4 |
| 11 | 19.8 | 15.9 | 23 | 27.4 | 5.5 | 35 | 39.8 | 3.5 |
| 12 | 20.6 | 10.7 | 24 | 29.1 | 21.3 | | | |

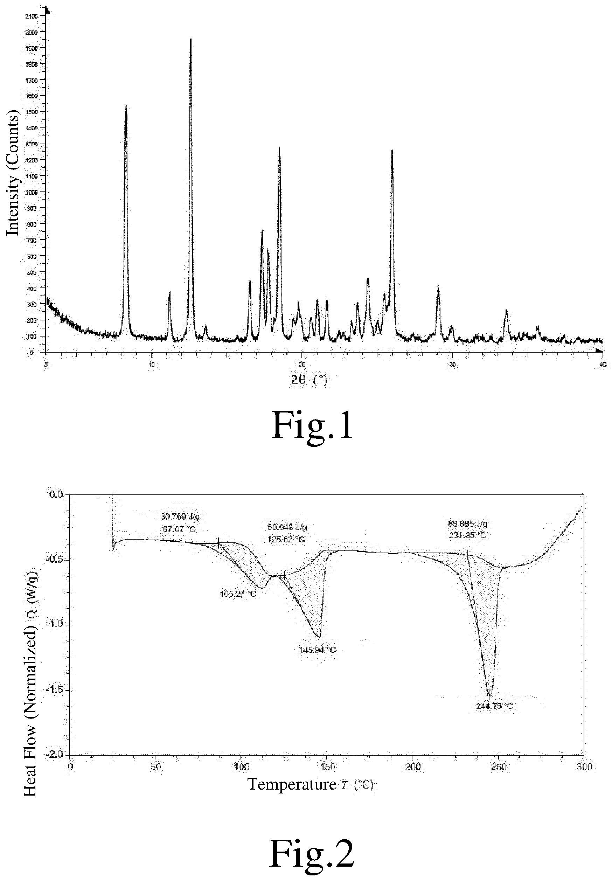

The crystalline form I has an XRPD pattern comprising peaks at diffraction angles (20) essentially the same as shown in Figure 1. Preferably, the XRPD pattern of crystalline form I is essentially the same as shown in Figure 1.

In more preferred embodiments, the crystalline form I has a DSC graph comprising endothermic peaks at about 105°C, about 146°C and about 245°C.

Preferably, the crystalline form I has a DSC graph comprising characteristic peaks essentially the same as shown in Figure 2. More preferably, the crystalline form I has a DSC graph essentially the same as shown in Figure 2.

In more preferred embodiments, in a thermogravimetric analysis, the crystalline form I has a weight loss of about 3.4% when heated to about 169°C.

The DSC graph of the crystalline form I has two consecutive endothermic peaks at 75-169°C, and the corresponding TGA graph shows a weight loss of about 3.4% before 169°C, indicating that the crystalline form I begins to lose water when the temperature is higher than 75°C.

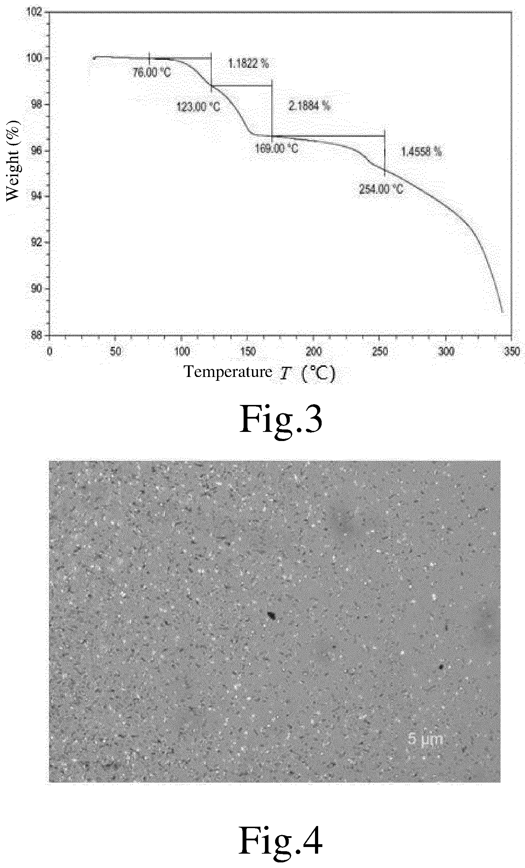

Preferably, the crystalline form I has a TGA graph essentially the same as shown in Figure 3.

Preferably, the crystalline form I has a scanning electron microscope image essentially the same as shown in Figure 4.

In some embodiments, the present invention provides a method for preparing the crystalline form I, comprising
adding a ketone solvent to compound A, wherein the ketone solvent preferably is a ketone having 3-6 carbon atoms, more preferably, acetone, butanone, methyl ethyl ketone, methyl isobutyl ketone and diethyl ketone,
stirring under heating, preferably heating to 40-80°C, more preferably, 50°C or 60°C,
and then adding water, wherein the volume ratio of the ketone solvent to water is preferably (1-15):1, preferably 10:1, so as to fully dissolve compound A;
then adding hydrochloric acid, wherein the concentration of hydrochloric acid is preferably 2-15 mol/L, preferably 12 mol/L, and the molar ratio of compound A to HCl is 1:(1-1.3),
stirring under heating, preferably heating to 40-80°C, more preferably, 50°C or 60°C,
filtering and optionally drying to obtain the crystalline form.

Disclosed herein is crystalline form II of compound A monohydrochloride dihydrate;
the crystalline form II has an XRPD pattern comprising characteristic peaks at diffraction angles (20) of about 9.9+0.2°, 13.2±0.2° and 16.2±0.2°;
preferably comprising characteristic peaks at diffraction angles (20) of about 9.9+0.2°, 11.0±0.2°, 13.2±0.2°, 13.5±0.2°, 13.8±0.2°, 16.2±0.2°, 19.4±0.2° and 25.2±0.2°;
most preferably comprising characteristic peaks at diffraction angles (2θ) of about 6.5±0.2°, 9.9±0.2°, 11.0±0.2°, 11.6±0.2°, 11.8±0.2°, 13.2±0.2°, 13.5±0.2°, 13.8±0.2°, 16.2±0.2°, 16.6±0.2°, 17.1±0.2°, 18.2±0.2°, 19.4±0.2°, 20.0±0.2°, 20.3±0.2°, 20.9±0.2°, 22.6±0.2°, 24.9±0.2°, 25.2±0.2° and 25.9±0.2°.

Preferably, the crystalline form II has an XRPD pattern comprising peaks at the following diffraction angles (20):

| **Peak No.** | **2θ(°) ± 0.2°** | **I%** | **Peak No.** | **2θ(°) ± 0.2°** | **I%** | **Peak No.** | **2θ(°) ± 0.2°** | **I%** |
|---|---|---|---|---|---|---|---|---|
| 1 | 6.5 | 16.1 | 14 | 18.2 | 10.9 | 27 | 29.2 | 7.7 |
| 2 | 8.1 | 9.5 | 15 | 19.4 | 22.9 | 28 | 30.0 | 7 |
| 3 | 9.9 | 100 | 16 | 20.0 | 11.5 | 29 | 30.4 | 7.6 |
| 4 | 11.0 | 24.6 | 17 | 20.3 | 16.9 | 30 | 31.8 | 4.7 |
| 5 | 11.6 | 13.3 | 18 | 20.9 | 15.4 | 31 | 32.8 | 6.9 |
| 6 | 11.8 | 17.4 | 19 | 22.6 | 12.2 | 32 | 33.5 | 6.3 |
| 7 | 13.2 | 32.6 | 20 | 23.8 | 7 | 33 | 34.1 | 8.1 |
| 8 | 13.5 | 26.8 | 21 | 24.9 | 15.7 | 34 | 35.0 | 5.3 |
| 9 | 13.8 | 20 | 22 | 25.2 | 18.4 | 35 | 35.9 | 5.5 |
| 10 | 15.4 | 7.5 | 23 | 25.9 | 12.3 | 36 | 36.8 | 5 |
| 11 | 16.2 | 29.9 | 24 | 26.8 | 8.4 | 37 | 39.3 | 5.9 |
| 12 | 16.6 | 14 | 25 | 27.1 | 8.2 | 38 | | |
| 13 | 17.1 | 12.9 | 26 | 27.9 | 7 | 39 | | |

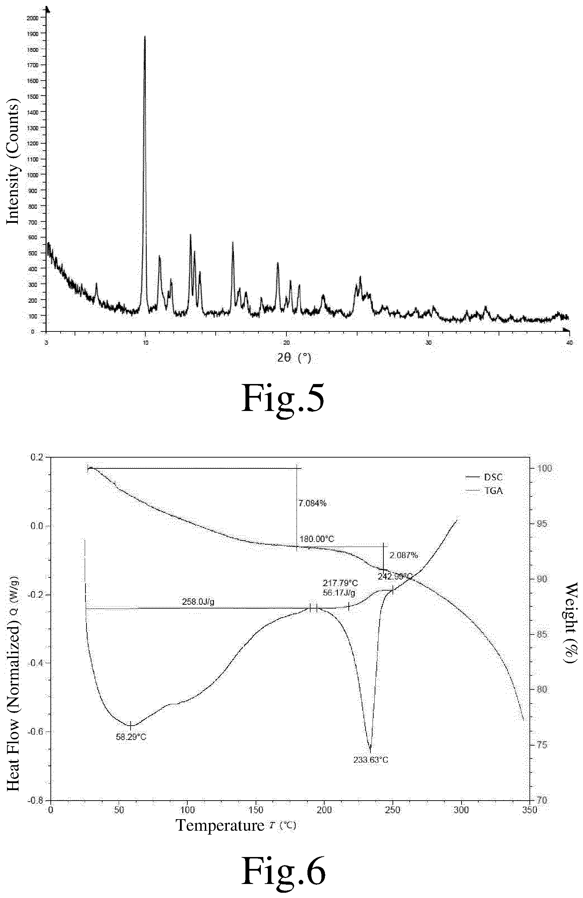

Preferably, the crystalline form II has an XRPD pattern comprising peaks at diffraction angles (20) essentially the same as shown in Figure 5. More preferably, the XRPD pattern of crystalline form II is essentially the same as shown in Figure 5.

Preferably, the crystalline form II has a DSC graph comprising a broad endothermic peak at about 25-180°C and an endothermic peak at about 217-243°C.

Preferably, in a thermogravimetric analysis, the crystalline form II has a weight loss of about 7.1% when heated to about 180°C.

The DSC graph of the crystalline form II has a broad endothermic peak at 25-180°C, and the corresponding TGA graph shows a weight loss of about 7.1% before 180°C, indicating that the crystalline form II begins to lose water when the temperature is higher than 25°C.

More preferably, the crystalline form II has a DSC-TGA graph essentially the same as shown in Figure 6.

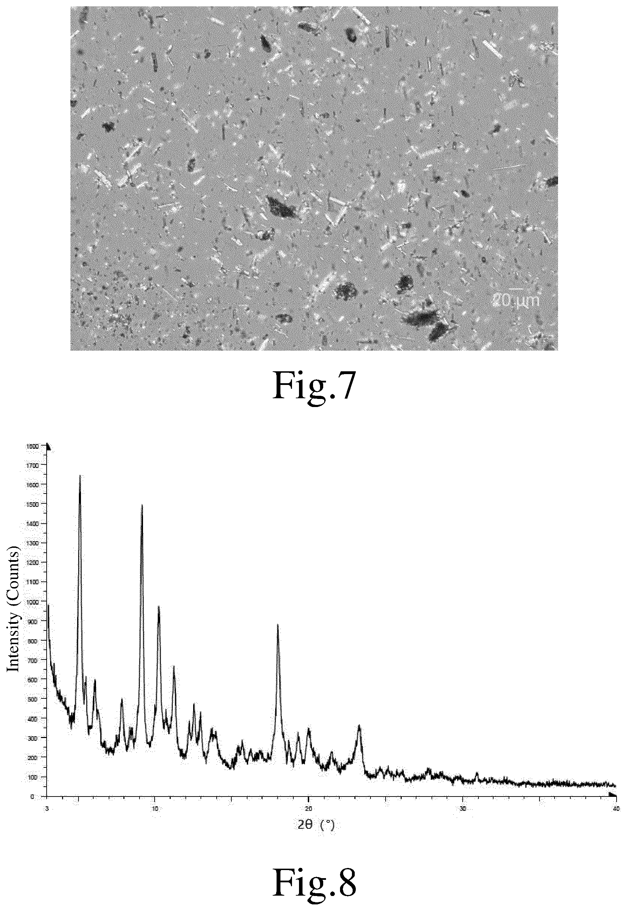

Preferably, the crystalline form II has a scanning electron microscope image essentially the same as shown in Figure 7.

The present disclosure provides a method for preparing the crystalline form II of compound A monohydrochloride dihydrate, comprising adding the crystalline form I of compound A monohydrochloride monohydrate to an aqueous alcoholic solvent having 1-10 carbon atoms (the alcoholic solvent is preferably an alcohol having 1-6 carbon atoms, including but not limited to methanol, ethanol, 1-propanol (n-propanol), 2-propanol (isopropanol), 1-butanol, 2-butanol and tert-butanol; the weight-to-volume ratio (g/mL) of the crystalline form I to the alcoholic solvent is about 1:(1-100), preferably 1:50), stirring the solution at a temperature of about 10-40°C (e.g., about 20-30°C) for about 10-60 hours (e.g., about 50 hours), filtering and optionally drying to obtain the crystalline form.

Disclosed herein is crystalline form III of compound A monohydrochloride sesquihydrate;
the crystalline form III has an XRPD pattern comprising characteristic peaks at diffraction angles (20) of about 5.1±0.2°, 9.1±0.2° and 10.2±0.2°;
preferably comprising characteristic peaks at diffraction angles (20) of about 5.1±0.2°, 5.4±0.2°, 6.0±0.2°, 9.1±0.2°, 10.2±0.2°, 11.2±0.2° and 18.0±0.2°;
most preferably comprising characteristic peaks at diffraction angles (20) of about 5.1±0.2°, 5.4±0.2°, 6.0±0.2°, 6.3±0.2°, 7.5±0.2°, 7.8±0.2°, 8.4±0.2°, 9.1±0.2°, 10.2±0.2°, 10.7±0.2°, 11.2±0.2°, 12.2±0.2°, 12.5±0.2°, 12.9±0.2°, 13.7±0.2°, 13.9±0.2°, 15.4±0.2°, 15.7±0.2°, 16.2±0.2°, 16.8±0.2°, 18.0±0.2°, 18.7±0.2°, 19.3±0.2°, 20.0±0.2°, 20.6±0.2°, 21.5±0.2° and 23.3±0.2°.

Preferably, the crystalline form III has an XRPD pattern comprising peaks at the following diffraction angles (20):

| **Peak No.** | **2θ(°) ± 0.2°** | **I%** | **Peak No.** | **2θ(°) ± 0.2°** | **I%** | **Peak No.** | **2θ(°) ± 0.2°** | **I%** |
|---|---|---|---|---|---|---|---|---|
| 1 | 5.1 | 100 | 12 | 12.2 | 23.2 | 23 | 19.3 | 19.7 |
| 2 | 5.4 | 34.8 | 13 | 12.5 | 28.5 | 24 | 20.0 | 20.9 |
| 3 | 6.0 | 36 | 14 | 12.9 | 26 | 25 | 20.6 | 11.2 |
| 4 | 6.3 | 26.5 | 15 | 13.7 | 18.8 | 26 | 21.5 | 13.5 |
| 5 | 7.5 | 18.8 | 16 | 13.9 | 19.8 | 27 | 23.3 | 21.7 |
| 6 | 7.8 | 30.1 | 17 | 15.4 | 15.5 | 28 | 24.6 | 8.9 |
| 7 | 8.4 | 19.7 | 18 | 15.7 | 17.2 | 29 | 25.2 | 9.1 |
| 8 | 9.1 | 90.7 | 19 | 16.2 | 13.9 | 30 | 26.1 | 7.9 |
| 9 | 10.2 | 59 | 20 | 16.8 | 14.1 | 31 | 27.8 | 8.5 |
| 10 | 10.7 | 26.8 | 21 | 18.0 | 52.7 | 32 | 31.0 | 7 |
| 11 | 11.2 | 40.3 | 22 | 18.7 | 16.6 | | | |

Preferably, the crystalline form III has an XRPD pattern comprising peaks at diffraction angles (20) essentially the same as shown in Figure 8. More preferably, the XRPD pattern of crystalline form III is essentially the same as shown in Figure 8.

Preferably, the crystalline form III has a DSC graph comprising a broad endothermic peak at about 25-134°C and endothermic peaks at about 137°C and about 251°C.

Preferably, in a thermogravimetric analysis, the crystalline form III has a weight loss of about 4.3% when heated to about 180°C.

The DSC graph of the crystalline form III has a broad endothermic peak and a small endothermic peak at 25-180°C, and the corresponding TGA graph shows a weight loss of about 4.3% before 180°C, indicating that the crystalline form III begins to lose water when the temperature is higher than 25°C.

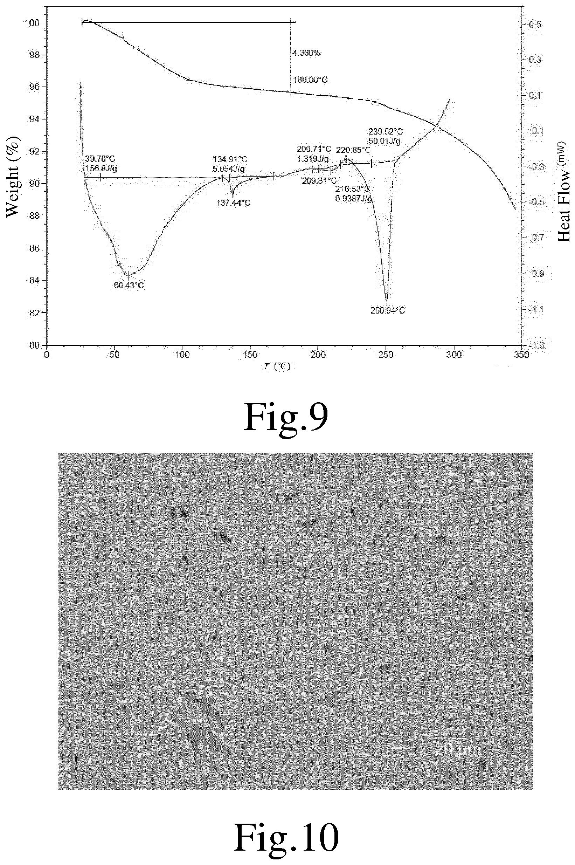

More preferably, the crystalline form III has a DSC-TGA graph essentially the same as shown in Figure 9.

Preferably, the crystalline form III has a scanning electron microscope image essentially the same as shown in Figure 10.

The present disclosure provides a method for preparing the crystalline form III of compound A monohydrochloride sesquihydrate, comprising adding the crystalline form I of compound A monohydrochloride monohydrate to an aqueous alcoholic solvent having 1-10 carbon atoms (the alcoholic solvent is preferably an alcohol having 1-6 carbon atoms, including but not limited to methanol, ethanol, 1-propanol (n-propanol), 2-propanol (isopropanol), 1-butanol, 2-butanol and tert-butanol; the weight-to-volume ratio (g/mL) of the crystalline form I to the alcoholic solvent is about 1:(1-100), preferably 1:50), adding an aromatic hydrocarbon solvent having 6-10 carbon atoms (e.g., toluene; the volume ratio of the alcoholic solvent to the aromatic hydrocarbon solvent is preferably about 1:(1-15), e.g., about 1:5) at a temperature of about 10-40°C (e.g., about 20-30°C), stirring the solution for about 1-6 hours (e.g., about 2 hours), filtering and optionally drying to obtain the crystalline form.

Disclosed herein is crystalline form IV of compound A monohydrochloride mono-dimethyl sulfoxide complex;
the crystalline form IV has an XRPD pattern comprising characteristic peaks at diffraction angles (20) of about 6.6+0.2°, 8.9±0.2° and 18.5±0.2°;
preferably comprising characteristic peaks at diffraction angles (20) of about 6.6±0.2°, 8.9±0.2°, 11.6±0.2°, 14.6±0.2°, 17.7±0.2°, 18.5±0.2°, 24.7±0.2° and 25.0±0.2°;
most preferably comprising characteristic peaks at diffraction angles (20) of about 4.1±0.2°, 6.6±0.2°, 8.9±0.2°, 11.2±0.2°, 11.6±0.2°, 13.3±0.2°, 13.8±0.2°, 14.6±0.2°, 15.7±0.2°, 17.7±0.2°, 18.1±0.2°, 18.5±0.2°, 19.4±0.2°, 20.0±0.2°, 20.7±0.2°, 21.4±0.2°, 22.0±0.2°, 22.6±0.2°, 23.0±0.2°, 23.8±0.2°, 24.7±0.2°, 25.0±0.2°, 26.5±0.2°, 27.9±0.2°, 29.7±0.2° and 32.8±0.2°.

Preferably, the crystalline form IV has an XRPD pattern comprising peaks at the following diffraction angles (20):

| **Peak No.** | **2θ(°) ± 0.2°** | **I%** | **Peak No.** | **2θ(°) ± 0.2°** | **I%** | **Peak No.** | **2θ(°) ± 0.2°** | **I%** |
|---|---|---|---|---|---|---|---|---|
| 1 | 4.1 | 26.8 | 10 | 17.7 | 54.3 | 19 | 23.0 | 22.9 |
| 2 | 6.6 | 64.5 | 11 | 18.1 | 28.6 | 20 | 23.8 | 18.1 |
| 3 | 8.9 | 71.3 | 12 | 18.5 | 100 | 21 | 24.7 | 38.1 |
| 4 | 11.2 | 18.4 | 13 | 19.4 | 23.6 | 22 | 25.0 | 38.6 |
| 5 | 11.6 | 55.5 | 14 | 20.0 | 26.2 | 23 | 26.5 | 17.9 |
| 6 | 13.3 | 18.4 | 15 | 20.7 | 27.8 | 24 | 27.9 | 21.6 |
| 7 | 13.8 | 19.4 | 16 | 21.4 | 21.1 | 25 | 29.7 | 16.8 |
| 8 | 14.6 | 39.4 | 17 | 22.0 | 24.8 | 26 | 32.8 | 10.9 |
| 9 | 15.7 | 21 | 18 | 22.6 | 26.5 | | | |

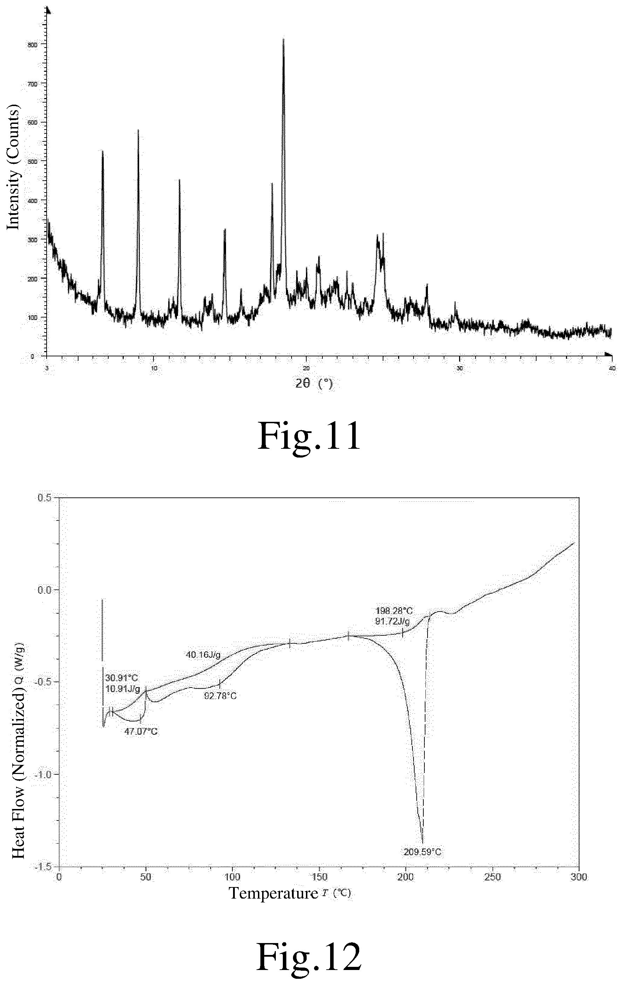

Preferably, the crystalline form IV has an XRPD pattern comprising peaks at diffraction angles (20) essentially the same as shown in Figure 11. More preferably, the XRPD pattern of crystalline form IV is essentially the same as shown in Figure 11.

Preferably, the crystalline form IV has a DSC graph comprising endothermic peaks at about 47°C, about 93°C and about 210°C.

Preferably, the crystalline form IV has a DSC graph comprising characteristic peaks essentially the same as shown in Figure 12. More preferably, the crystalline form IV has a DSC graph essentially the same as shown in Figure 12.

Preferably, in a thermogravimetric analysis, the crystalline form IV has a weight loss of about 14.1% when heated to about 217°C.

The DSC graph of the crystalline form IV has an endothermic peak at 30-217°C, and the corresponding TGA graph shows a weight loss of about 14.1% before 217°C, indicating that the crystalline form IV begins to lose the solvent (DMSO) when the temperature is higher than 30°C.

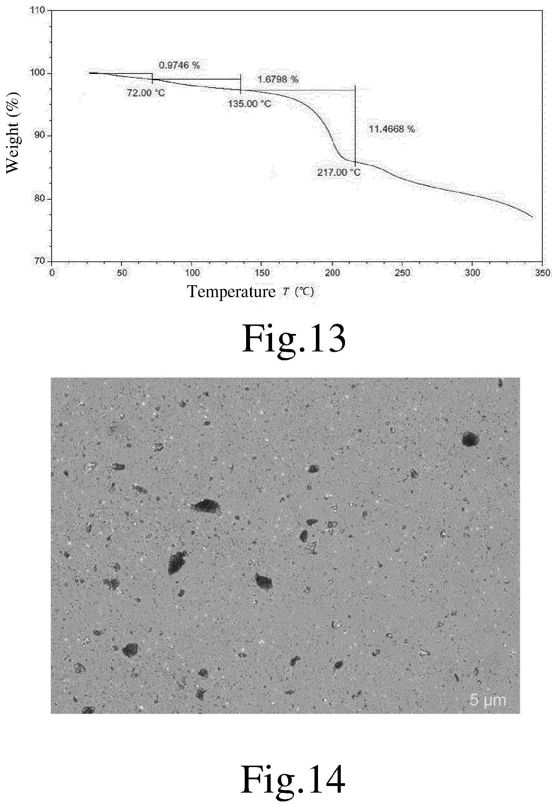

Preferably, the crystalline form IV has a TGA graph essentially the same as shown in Figure 13.

Preferably, the crystalline form IV has a scanning electron microscope image essentially the same as shown in Figure 14.

The present disclosure provides a method for preparing crystalline form IV of compound A monohydrochloride mono-dimethyl sulfoxide complex, comprising adding the crystalline form I of compound A monohydrochloride monohydrate to dimethyl sulfoxide (the weight-to-volume ratio (g/mL) of the crystalline form I to the dimethyl sulfoxide is about 1:(1-100), preferably about 1:50), adding an aromatic hydrocarbon solvent having 6-10 carbon atoms (e.g., toluene; the volume ratio of the dimethyl sulfoxide to the aromatic hydrocarbon solvent is preferably about 1:(1-15), e.g., about 1:9) at a temperature of about 10-40°C (e.g., about 20-30°C), stirring the solution for about 1-6 hours (e.g., about 2 hours), filtering and optionally drying to obtain the crystalline form.

### Pharmaceutical composition, therapeutic method and use thereof

In some embodiments, the present invention provides a pharmaceutical composition comprising the crystalline form I, and one or more pharmaceutically acceptable carriers.

In some embodiments, the present invention provides crystalline form I for use in the prevention or treatment of a disease mediated by the Rho-associated protein kinase, wherein the disease is selected from:
a cardiovascular disorder, wherein the cardiovascular disorder is hypertension, atherosclerosis, restenosis, cardiac hypertrophy, cerebral ischemia, cerebral vasospasm, or erectile dysfunction;
inflammation, wherein the inflammation is asthma, cardiovascular inflammation, ulcerative colitis, or renal inflammation;
a central nervous system disorder, wherein the central nervous system disorder is neuronal degeneration or spinal cord injury; and the central nervous system disorder is preferably Huntington's disease, Parkinson's disease, Alzheimer's disease, Amyotrophic lateral sclerosis, or multiple sclerosis;
an arterial thrombotic disorder, wherein the arterial thrombotic disorder is platelet aggregation, or leukocyte aggregation;
a neoplastic disease, wherein the neoplastic disease is a lymphoma, carcinoma, leukemia, astrocytoma, soft tissue sarcoma, sarcoma, or blastoma, and the carcinoma is squamous cell cancer, small-cell lung cancer, pituitary cancer, esophageal cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, bladder cancer, liver cancer, breast cancer, colon cancer, colorectal cancer, endometrial or uterine carcinoma, salivary gland carcinoma, kidney cancer, prostate cancer, vulval cancer, thyroid cancer, brain cancer, endometrial cancer, testis cancer, cholangiocarcinoma, gallbladder carcinoma, gastric cancer, melanoma, or head and neck cancer;
a metabolic syndrome;
insulin resistance;
hyperinsulinemia;
type 2 diabetes;
glucose intolerance;
osteoporosis; and
an ocular disorder, wherein the ocular disorder is ocular hypertension, age related macular degeneration, choroidal neovascularization, diabetic macular edema, iris neovascularization, uveitis, glaucoma, or retinitis of prematurity, and the glaucoma is primary open-angle glaucoma, acute angle-closure glaucoma, pigmentary glaucoma, congenital glaucoma, normal tension glaucoma, secondary glaucoma or neo vascular glaucoma.

In some embodiments, the present invention provides crystalline form I for use in the prevention or treatment of an autoimmune disorder, wherein the autoimmune disorder is selected from rheumatoid arthritis, systemic lupus erythematosus, psoriasis, Crohn's disease, atopic dermatitis, eczema, and graft-versus-host disease.

In some embodiments, the present invention provides crystalline form I for use in the prevention or treatment of a fibrotic disorder, wherein the fibrotic disorder is selected from liver fibrosis, lung fibrosis, and kidney fibrosis.

In some embodiments, the present invention provides crystalline form I for use in the prevention or treatment of a disease mediated by the Rho-associated protein kinase, wherein the disease is selected from lupus nephritis, atherosclerosis, rheumatoid arthritis, hemangioma, angiofibroma, lung fibrosis, psoriasis, corneal graft rejection, insulin-dependent diabetes mellitus, multiple sclerosis, myasthenia gravis, Chron's disease, autoimmune nephritis, primary biliary cirrhosis, acute pancreatitis, allograph rejection, allergic inflammation, contact dermatitis, delayed hypersensitivity, inflammatory bowel disease, septic shock, osteoporosis, osteoarthritis, neuronal inflammation, Osier-Weber syndrome, restenosis, fungal infection, parasitic infection, and viral infection.

The present disclosure provides a pharmaceutical composition comprising any one or more of the salt of compound A, and its crystalline forms I, II, III and IV, and one or more pharmaceutically acceptable carriers.

The present disclosure provides use of any one or more of the salt of compound A, and its crystalline forms I, II, III and IV in the manufacture of a medicament as a Rho-associated protein kinase (ROCK) inhibitor, preferably selective ROCK2 inhibitor.

The present disclosure provides any one or more of the salt of compound A, and its crystalline forms I, II, III and IV for use as a Rho-associated protein kinase (ROCK) inhibitor, preferably selective ROCK2 inhibitor.

The present disclosure provides a method for the prophylaxis or treatment of a disease mediated by the Rho-associated protein kinase (ROCK), comprising administering to a subject in need thereof, preferably a mammal, a prophylactically or therapeutically effective amount of any one or more of the salt of compound A, and its crystalline forms I, II, III and IV.

The disease mediated by the Rho-associated protein kinase (ROCK) includes an autoimmune disorder (comprising rheumatoid arthritis, systemic lupus erythematosus (SLE; lupus), psoriasis, Crohn's disease, atopic dermatitis, eczema, or graft-versus-host disease (GVHD)); a cardiovascular disorder (comprising hypertension, atherosclerosis, restenosis, cardiac hypertrophy, cerebral ischemia, cerebral vasospasm, or erectile dysfunction); inflammation (comprising asthma, cardiovascular inflammation, ulcerative colitis, or renal inflammation); a central nervous system disorder (comprising neuronal degeneration or spinal cord injury; and the central nervous system disorder is preferably Huntington's disease, Parkinson's disease, Alzheimer's disease, Amyotrophic lateral sclerosis (ALS), or multiple sclerosis); an arterial thrombotic disorder (comprising platelet aggregation, or leukocyte aggregation); a fibrotic disorder (comprising liver fibrosis, lung fibrosis, or kidney fibrosis); a neoplastic disease (comprising a lymphoma, carcinoma (e.g., squamous cell cancer, small-cell lung cancer, pituitary cancer, esophageal cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, bladder cancer, liver cancer, breast cancer, colon cancer, colorectal cancer, endometrial or uterine carcinoma, salivary gland carcinoma, kidney cancer, prostate cancer, vulval cancer, thyroid cancer, brain cancer, endometrial cancer, testis cancer, cholangiocarcinoma, gallbladder carcinoma, gastric cancer, melanoma, or head and neck cancer), leukemia, astrocytoma, soft tissue sarcoma, sarcoma, or blastoma); a metabolic syndrome; insulin resistance; hyperinsulinemia; type 2 diabetes; glucose intolerance; osteoporosis; an ocular disorder (comprising ocular hypertension, age related macular degeneration (AMD), choroidal neovascularization (CNV), diabetic macular edema (DME), iris neovascularization, uveitis, glaucoma (comprising primary open-angle glaucoma, acute angle-closure glaucoma, pigmentary glaucoma, congenital glaucoma, normal tension glaucoma, secondary glaucoma or neo vascular glaucoma), or retinitis of prematurity (ROP)).

The disease mediated by the Rho-associated protein kinase (ROCK) includes lupus nephritis, atherosclerosis, rheumatoid arthritis (RA), hemangioma, angiofibroma, lung fibrosis, psoriasis, corneal graft rejection, insulin-dependent diabetes mellitus, multiple sclerosis, myasthenia gravis, Chron's disease, autoimmune nephritis, primary biliary cirrhosis, acute pancreatitis, allograph rejection, allergic inflammation, contact dermatitis, delayed hypersensitivity, inflammatory bowel disease, septic shock, osteoporosis, osteoarthritis, neuronal inflammation, Osier-Weber syndrome, restenosis, fungal infection, parasitic infection, and viral infection.

As used herein, the term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the present invention includes, but is not limited to sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g., Remington's Pharmaceutical Sciences (1990).

The composition of the present invention can act systemically and/or topically. To this end, it can be administered through a suitable route, such as through injection, intravenous, intraarterial, subcutaneous, intraperitoneal, intramuscular, or transdermal administration, or administered via oral, buccal, nasal, transmucosal, topical, as an ophthalmic formulation, or via inhalation.

For these routes of administration, the composition of the present invention can be administered in a suitable dosage form.

The dosage form may be solid, semi-solid, liquid, or gas formulations, specifically including, but not limited to, tablets, capsules, powders, granules, lozenges, hard candies, powders, sprays, creams, salves, suppositories, gels, pastes, lotions, ointments, aqueous suspensions, injectable solutions, suspensions, elixirs, and syrups.

The pharmaceutical composition of the present invention may be manufactured by any process well known in the art, e.g., by means of mixing, dissolving, granulating, dragee-making, levigating, emulsifying, lyophilizing processes, or the like.

As used herein, the term "therapeutically effective amount" refers to the amount of the compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition is about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, and 25 mg, *etc.*

Unless otherwise indicated, the term "treating" or "treatment", as used herein, means reversing, alleviating, inhibiting the progress of, or preventing a disorder, condition, or disease to which such term applies, or one or more symptoms of such disorder, condition, or disease.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

### Example

The present invention is explained in more detail below with reference to the examples.

Unless otherwise specified, the starting materials and reagents employed in the following Examples are all commercially available products or can be prepared through known methods.

The detection instruments and conditions used in the following examples are as follows:
(1) X-ray powder diffraction (XRPD)
   (a) Instrument Model: Bruker D8 advance, equipped with a LynxEye detector
      Test conditions: the anode target material was copper, the light pipe was set to (40KV 40mA), the 2θ scan angle for the sample was from 3° to 40°, and scan step was 0.02°.
   (b) Instrument Model: Bruker D2 phaser
   Test conditions: the anode target material was copper, the light pipe was set to (30KV 10mA), the 2θ scan angle for the sample was from 4° to 50°, and scan step was 0.02°.
(2) differential scanning calorimetry analysis (DSC)
   Instrument Model: (a) TA Discovery DSC 250 (TA Instruments, US); (b) TA Discovery DSC 25 (TA Instruments, US)
   Test conditions: the heating rate was 10°C/min, and dry nitrogen was used as the purge gas.
(3) thermogravimetric analysis (TGA)
   Instrument Model: (a) Discovery TGA 55 (TA Instruments, US); (b) TGA 4000 (PerkinElmer, Germany)
   Test conditions: automatic weighing in the heating furnace, the heating rate was 10°C/min, and dry nitrogen was used as the purge gas.
(4) polarizing microscope analysis (PLM)
   Instrument Model: Polarizing Microscope ECLIPSE LV100POL (Nikon, JPN)
(5) Nuclear magnetic resonance (¹H NMR)
   Instrument Model: Bruker Advance 400, equipped with B-ACS 120 automatic sampling system
(6) Dynamic vapour sorption analysis (DVS)
   Instrument Model: DVS Intrinsic (SMS, UK)
   Test conditions: a gradient mode was used, the humidity ranged from 0% to 90%, the humidity increment of each gradient was 10%, and the holding time of each gradient was 1h.

### Example 1: preparation of (6-(4-((4-(1H-pyrazol-4-yl)phenyl)amino)pyrimidin-2-yl)-1-methyl-1H-indol-2-yl)(3,3-difluoroazetidin-1-yl)methanone (Compound A) (according to PCT/CN2018/093713)

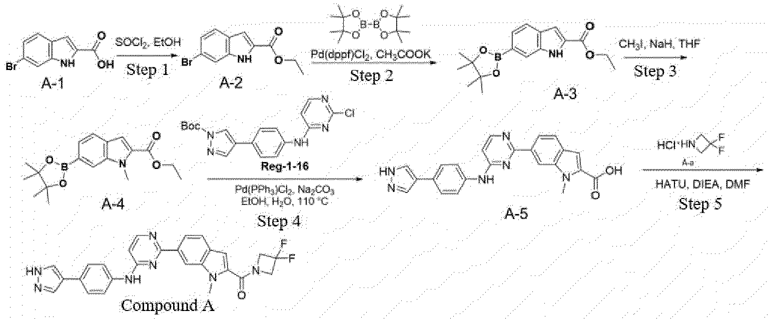

### Step 1:

Compound A-1 (20 g, 83.31 mmol) and ethanol (200 mL) were added to a 500 mL flask, thionyl chloride (19.82 g, 166.63 mmol) was added, and then the reaction was performed at 60°C for 3 hours. Thin layer chromatography (petroleum ether / ethyl acetate =10:1) assay indicated the reaction was complete. The reaction solution was concentrated to afford a crude product, which was dissolved in dichloromethane (500 mL), and the resulting solution was washed twice with a saturated aqueous solution of sodium bicarbonate (150 mL each). The organic phase was washed with saturated brine, then dried over anhydrous sodium sulfate, filtered, and concentrated to afford compound **A-2** (21 g, brown solid, yield: 94.01%). MS *m*/*z* (ESI): 266.1; 268.1 [M-H].

### Step 2:

Compound **A-2** (21 g, 78.33 mmol) and bis(pinacolato)diboron (26.85 g, 105.74 mmol) were dissolved in 1,4-dioxane (200 mL), potassium acetate (23.06 g, 234.98 mmol) and Pd(dppf)Cl₂ (3.24 g, 3.91 mmol) were added, purge with argon was performed for 3 times, and the reaction was placed in an oil bath at 80 °C overnight. LC-MS indicated the reaction was complete. The reaction solution was cooled to room temperature, concentrated under reduced pressure, and the residue was purified by column chromatography (petroleum ether / ethyl acetate = 100:1 to 5:1) to afford compound **A-3** (17.5 g, white solid, yield: 70.89%).

¹H NMR (400 MHz, CDCl₃) *δ* 8.92 (s, 1H), 7.92 (s, 1H), 7.69 (d, *J* = 8.1 Hz, 1H), 7.57 (d, *J* = 8.1 Hz, 1H), 7.22 - 7.18 (m, 1H), 4.42 (q, *J =* 7.1 Hz, 2H), 1.42 (t, *J =* 7.1 Hz, 3H), 1.37 (s, 12H). MS *m*/*z* (ESI): 316.2 [M+H].

### Step 3:

Compound **A-3** (10.0 g, 31.8 mmol) was dissolved in tetrahydrofuran (250 mL), sodium hydride (1.91 g, 47.8 mmol) was added under ice bath cooling, and then the reaction was performed for 30 minutes. Iodomethane (13.5 g, 95.4 mmol) was slowly added to the reaction solution, and the reaction was performed at room temperature overnight. Thin layer chromatography (petroleum ether / ethyl acetate = 5:1) indicated the reaction was complete. The reaction solution was quenched with water (100 mL), and extracted with ethyl acetate (150 mL x 2). The combined organic phases were washed sequentially with a saturated aqueous solution of ammonium chloride (200 mL x 2) and saturated brine (300 mL x 2), dried over anhydrous sodium sulfate, filtered, and concentrated. The crude product was separated and purified by column chromatography (petroleum ether / ethyl acetate = 15:1) to afford compound **A-4** (6.5 g, yellow solid, yield: 62.5%).

¹H NMR (400 MHz, CDCl₃) *δ* 7.91 (s, 1H), 7.68 - 7.65 (m, 1H), 7.57 (d, *J* = 8.0 Hz, 1H), 7.27 (s, 1H), 4.12 (s, 3H), 3.91 (s, 3H), 1.38 (s, 12H).

### Step 4:

Compound **Reg-1-16** (1.00 g, 2.70 mmol) and **A-4** (1.33 g, 4.04 mmol) were dissolved in a mixed solution of ethanol/water (8:1) (120 mL), sodium carbonate (572 mg, 5.40 mmol) and Pd(PPh₃)Cl, (189 mg, 0.27 mmol) were added, purge with argon was performed for 3 times, and the reaction was placed in an oil bath at 110 °C overnight. LC-MS indicated the reaction was complete. The reaction solution was cooled to room temperature, and concentrated under reduced pressure. The residue was diluted with water (30 mL), and the pH was adjusted to 1 with 6N HCl. A large amount of solid precipitated, and was filtered. The solide was wahsed with methanol to afford compound **A-5** (900 mg, yellow solid, crude product).

¹H NMR (400 MHz, DMSO-*d₆*) *δ* 11.54 (s, 1H), 8.77 (s, 1H), 8.38 (d, *J* = 7.2 Hz, 1H), 8.14 (s, 2H), 8.03 (d, *J =* 8.8 Hz, 1H), 7.92 (d, *J =* 8.4 Hz, 1H), 7.80 - 7.69 (m, 4H), 7.33 (s, 1H), 7.07 (d, *J* = 8.0 Hz, 1H), 4.16 (s, 3H).

### Step 5:

Compound **A-5** (300 mg, 0.73 mmol) was dissolved in N,N-dimethylformamide (6 mL), HATU (335 mg, 0.88 mmol) and DIEA (377 mg, 2.92 mmol) were added, and the reaction was performed at room temperature for 30 min. Then, compound **A-a** (114 mg, 0.88 mmol) was added, and the reaction was continued at room temperature for 2 hours. LC-MS indicated the reaction was complete. The reaction solution was concentrated under reduced pressure, and the crude product was purified by high performance liquid chromatography to afford compound **A** (185 mg, yellow solid, yield: 52.1%).

¹H NMR (400 MHz, DMSO-*d₆*) *δ* 10.63 (s, 1H), 8.52 (s, 1H), 8.41 (d, *J =* 6.4 Hz, 1H), 8.09 (s, 2H), 8.06 (dd, *J =* 8.4, 1.2 Hz, 1H), 7.83 (d, *J =* 8.4 Hz, 1H), 7.77 (d, *J =* 8.0 Hz, 2H), 7.72 (d, *J =* 8.4 Hz, 2H), 7.13 (s, 1H), 6.86 (d, *J =* 6.4 Hz, 1H), 4.91 (s, 2H), 4.57 (s, 2H), 4.05 (s, 3H). MS *m*/*z* (ESI): 486.2 [M+H].

### Example 2: preparation of crystalline form I of compound A monohydrochloride monohydrate

Compound A (3.7 g) was added to a reaction flask, acetone (250 ml) was added, the reaction solution was heated to 50°C and stirred for 10 min, the solid completely dissolved. Water (26.7 ml) was added, and the mixture was stirred to get a clear solution. Concentrated hydrochloric acid (12 mol/L) (0.641 ml) was added, and a large amount of solids precipitated. The mixture was stirred at 50°C for 18h, and filtered. The solid was collected, and dried under vacuum at 50°C for 8h to obtain a crystalline form (2.5 g, yield: 60.8%). The XRPD pattern obtained by X-ray powder diffraction analysis is shown in Figure 1; upon DSC and TGA analysis, the DSC graph is shown in Figure 2, and the TGA graph is shown in Figure 3; the sample was examined through a scanning electron microscope, and the crystal morphology is shown in Figure 4.

¹H NMR (400 MHz, DMSO-*d₆*) δ: 11.19 (s, 1H), 8.68 (s, 1H), 8.39 (d, J=6.8Hz, 1H), 8.11 (s, 2H), 8.04 (d, J=8Hz, 1H), 7.86 (d, J=8Hz, 1H), 7.78-7.73 (m, 4H), 7.14 (s, 1H), 6.99 (d, J=6.4Hz, 1H), 4.99-4.57 (brs, 4H), 4.07 (s, 3H).

### Reference Example 3: preparation of crystalline form II of compound A monohydrochloride dihydrate

The crystalline form I of compound A monohydrochloride monohydrate (2.0g) was added to methanol (100 ml, water content: 0.08%), the mixture was stirred at 20-30°C for 48h, and filtered to collect the solid, which was dried under vacuum at 50°C for 8 h, to obtain a crystalline form (1.0 g, yield: 48.4%). The XRPD pattern obtained by X-ray powder diffraction analysis is shown in Figure 5; the DSC-TGA graph obtained by the DSC and TGA analysis is shown in Figure 6; the sample was examined through a scanning electron microscope, and the crystal morphology is shown in Figure 7.

¹H NMR (400 MHz, DMSO-*d*₆) δ: 11.18 (s, 1H), 8.65 (s, 1H), 8.39 (d, J=6.8Hz, 1H), 8.10 (s, 2H), 8.04 (d, J=8Hz, 1H), 7.86 (d, J=8Hz, 1H), 7.778-7.73 (m, 4H), 7.14 (s, 1H), 6.97 (d, J=6.4Hz, 1H), 4.91-4.57 (brs, 4H), 4.06 (s, 3H).

### Reference Example 4: preparation of crystalline form III of compound A monohydrochloride sesquihydrate

The crystalline form I of compound A monohydrochloride monohydrate (2.0 g) was added to methanol (100 ml, water content: 0.08%), the temperature was maintained at 20-30°C, toluene (560 ml) was added dropwise, and a solid precipitated. The mixture was stirred for 2 h, and filtered to collect the solid, which was dried under vacuum at 50°C for 8 h, to obtain a crystalline form (1.1 g, yield: 54.2%). The XRPD pattern obtained by X-ray powder diffraction analysis is shown in Figure 8; the DSC-TGA graph obtained by the DSC and TGA analysis is shown in Figure 9; the sample was examined through a scanning electron microscope, and the crystal morphology is shown in Figure 10.

¹H NMR (400 MHz, DMSO-*d₆*) δ:10.98 (s, 1H), 8.61 (s, 1H), 8.37 (d, J=6.8Hz, 1H), 8.10 (s, 2H), 8.04 (d, J=8Hz, 1H), 7.85 (d, J=8Hz, 1H), 7.77-7.72 (m, 4H), 7.13 (s, 1H), 6.94 (d, J=6.4Hz, 1H), 4.91-4.57 (brs, 4H), 4.05 (s, 3H).

### Reference Example 5: preparation of crystalline form IV of compound A monohydrochloride mono-dimethyl sulfoxide complex

The crystalline form I of compound A monohydrochloride monohydrate (2.0 g) was added to dimethyl sulfoxide (100 ml), the temperature was maintained at 20-30°C, toluene (900 ml) was added dropwise, and a solid precipitated. The mixture was stirred for 2 h, and filtered to collect the solid, which was dried under vacuum at 50°C for 8 h, to obtain a crystalline form (0.8 g, yield: 36.0%). The XRPD pattern obtained by X-ray powder diffraction analysis is shown in Figure 11; upon DSC and TGA analysis, the DSC graph is shown in Figure 12, and the TGA graph is shown in Figure 13; the sample was examined through a scanning electron microscope, and the crystal morphology is shown in Figure 14.

¹H NMR (400 MHz, DMSO-*d*₆) δ: 11.06 (s, 1H), 8.64 (s, 1H), 8.37 (d, J=6.8Hz, 1H), 8.10 (s, 2H), 8.04 (d, J=8Hz, 1H), 7.85 (d, J=8Hz, 1H), 7.77-7.72 (m, 4H), 7.13 (s, 1H), 6.94 (d, J=6.4Hz, 1H), 4.95-4.57 (brs, 4H), 4.06 (s, 3H), 2.36 (s, 6H).

### Experimental Example

### Experimental Example 1: pharmacokinetic test

The crystalline form I of compound A monohydrochloride monohydrate and compound A (vehicles for both samples were 0.5% CMC-Na) were respectively orally administered once to two groups of beagle dogs at a dose of 20 mpk, and blood samples were collected before the administration (0h) and at 0.25, 0.5, 1, 2, 4, 6, 8 and 24 hours after the administration. The samples were processed and subjected to a chromatographic analysis. The test results are shown in the table below:

| **Drug** | **Cₘₐₓ (ng/mL)** | **AUC₀₋ₗₐₛₜ (ng•h/mL)** |
|---|---|---|
| Crystalline form I of compound A monohydrochloride monohydrate | 3856.987 | 19433.501 |
| Compound A | 146.605 | 521.765 |

According to the above results, the exposure upon oral administration of crystalline form I of compound A monohydrochloride monohydrate is significantly better than that of compound A.

### Experimental Example 2: solid stability test

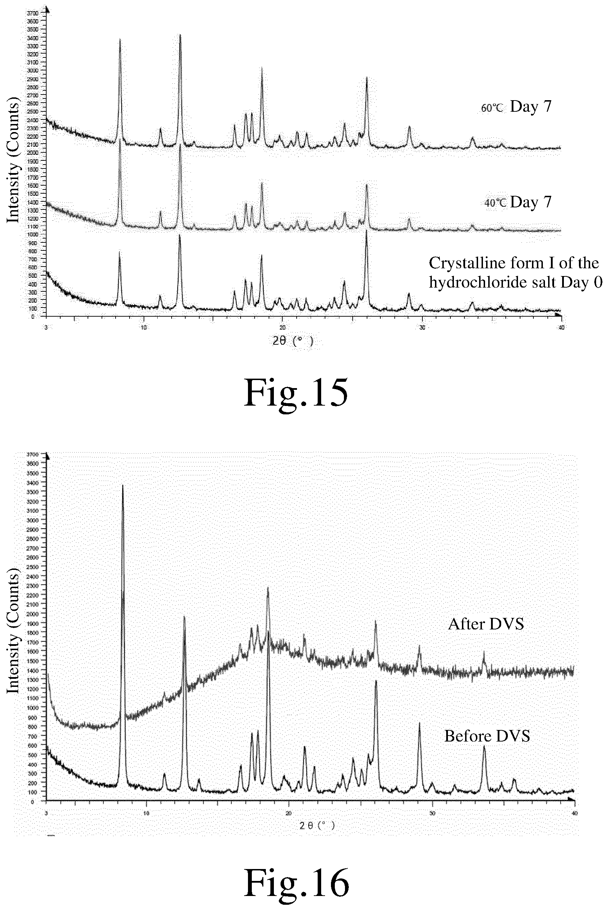

Crystalline form I of compound A monohydrochloride monohydrate was placed under the conditions of 40°C/75% RH and 60°C/90% RH for 7 days, respectively, and was subjected to X-ray powder diffraction analysis. The test results are shown in Figure 15. The results showed that the crystalline form I of compound A monohydrochloride monohydrate did not change.

### Experimental Example 3: solubility test

Crystalline form I of compound A monohydrochloride monohydrate was tested for solubility in a simulated gastric fluid (SGF), fed state simulated intestinal fluid (FeSSIF), and fasted state simulated intestinal fluid (FaSSIF), respectively. The test results are as follows:
SGF: 0.0012 mg/ml;
FeSSIF: 0.017 mg/ml;
FaSSIF: 0.00028 mg/ml.

### Experimental Example 4: DVS test

The dynamic moisture adsorption-desorption curve of the crystalline form I of compound A monohydrochloride monohydrate was determined using DVS Intrinsic (SMS, UK) in a gradient mode with a humidity range of 0% to 90% (the humidity increment was 10% for each gradient, and each gradient was maintained for 1h), and the crystalline form of the sample before and after the DVS test was tested by XRPD. DVS indicated that the crystalline form I of compound A monohydrochloride monohydrate had a water absorption of 0.67% at 0-10% RH, and a water absorption of 0.9% at 10-90% RH; the XRPD patterns of the sample before and after the DVS test are shown in Figure 16. According to Figure 16, the crystalline form I of compound A monohydrochloride monohydrate did not change after the DVS test.

### Experimental Example 5: crystalline form transformation test

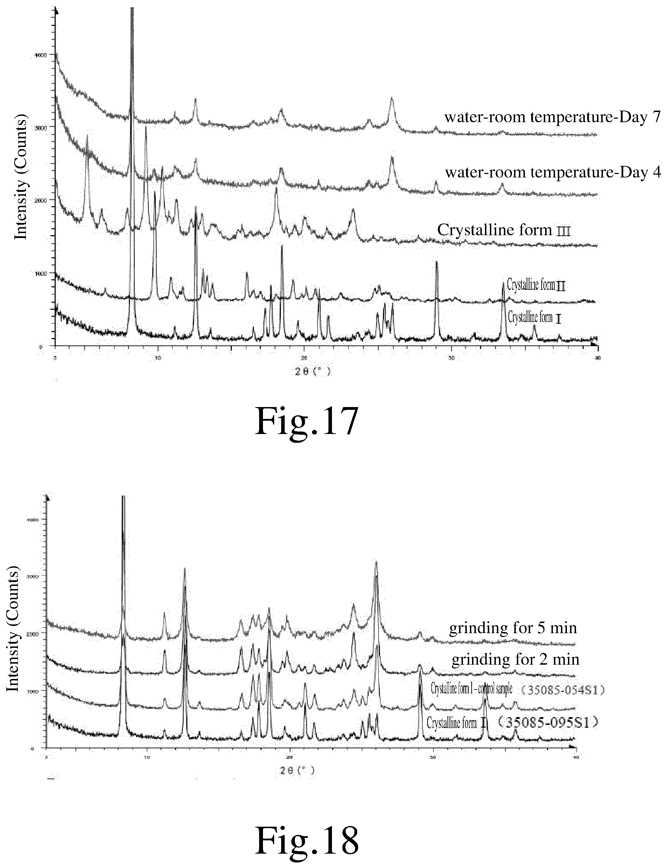

About 5 mg of each of crystalline form I, crystalline form II and crystalline form III were mixed and placed in a 4 mL vial, and then 0.8 mL of water was added. The suspension was stirred at room temperature for 7 days. The mixture was subjected to X-ray powder diffraction analysis on Day 4 and Day 7, and the results are shown in Figure 17. The results showed that on Day 4, the mixture transformed as a mixed crystal of crystalline form I and crystalline form II, and crystalline form III disappeared; on Day 7, the mixture completely transformed as crystalline form I. This indicates that the most stable crystalline form in water at room temperature is crystalline form I.

### Experimental Example 6: grinding test

An appropriate amount of crystalline form I of compound A monohydrochloride monohydrate was taken and ground for 2 minutes and 5 minutes, respectively, and subjected to an X-ray powder diffraction analysis. The test results are shown in Figure 18. The results indicates that the crystalline form did not change.

### Experimental Example 7: solid stability test

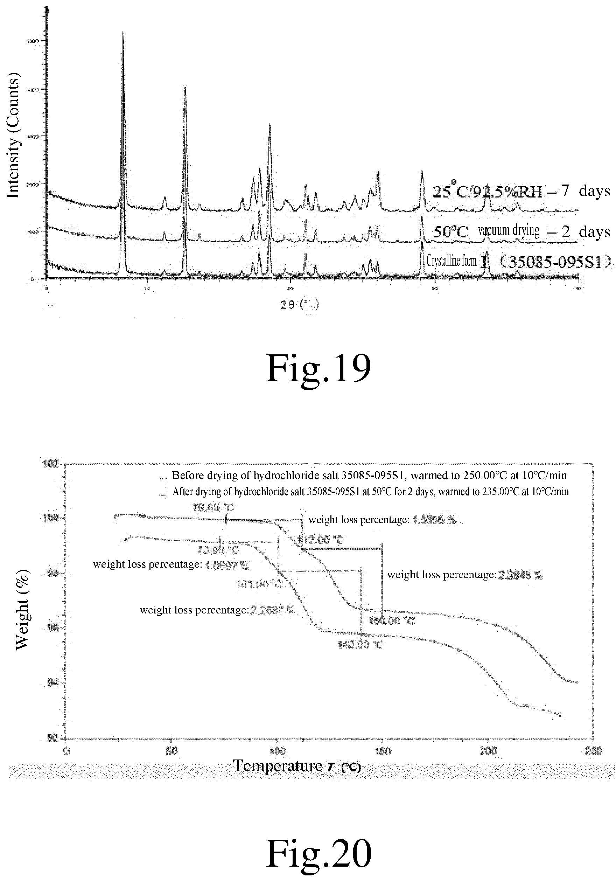

20.8 mg of crystalline form I of compound A monohydrochloride monohydrate was taken and placed in a 4 mL vial. The vial was left open, and dried under vacuum at 50°C for 2 days. The sample was subjected to X-ray powder diffraction and TGA analyses, and the results are shown in Figure 19 and Figure 20.

19.3 mg of crystalline form I of compound A monohydrochloride monohydrate was taken and placed in a 4 mL vial. The vial was left open, and placed under a condition of 25°C/92.5% RH for 7 days. The sample was subjected to X-ray powder diffraction analysis, and the results are shown in Figure 19.

Results in Figure 19 and Figure 20 indicated that the physical stability of crystalline form I of compound A monohydrochloride monohydrate was good, and the crystalline form did not change. The TGA weight loss of the sample after drying did not change, indicating that the water molecules and the drug molecules are firmly associated in crystalline form I.

### Experimental Example 8: determination of the chloride ion content in the sample by potentiometric titration

270 mg of crystalline form I of compound A monohydrochloride monohydrate was taken and placed in a titration beaker, 25 mL of dimethyl sulfoxide was firstly added to dissolve, then 15 mL of water was added, and the solution was stirred thoroughly. The titration was performed by Mettler T50 potentiometric titrator with DMI141-SC silver electrode and silver nitrate titration solution (0.1 mol/L), and the chloride ion content in the sample was calculated according to the consumption of the silver nitrate titration solution. The chloride ion content in multiple batches of the samples as determined by this method was between 6.5% and 6.7%, while the theoretical content of chloride ion in compound A monohydrochloride monohydrate is 6.6%. As such, the measured value is consistent with the theoretical value.

| **Batch** | **Chloride ion content (%)** |
|---|---|
| 1 | 6.5% |
| 2 | 6.7% |
| 3 | 6.5% |
| 4 | 6.5% |
| 5 | 6.6% |

### Experimental Example 9: determination of the water content in the sample by a Karl Fischer method

50 mg of crystalline form I of compound A monohydrochloride monohydrate was taken and added to a Karl Fischer sample bottle. The sample bottle was sealed, and heated to 160 °C. The water content was determined on a Metrohm KF 831 Coulometer through a coulometric heating furnace method. The water content in multiple batches of the samples as determined by this method was between 3.2% and 4.0%, while the theoretical water content of compound A monohydrochloride monohydrate is 3.3%. As such, the measured value is consistent with the theoretical value.

| **Batch** | **Water content (%)** |
|---|---|
| 1 | 3.6% |
| 2 | 3.7% |
| 3 | 3.5% |
| 4 | 4.0% |
| 5 | 3.2% |

## Claims

1. A crystalline form I of compound A monohydrochloride monohydrate, 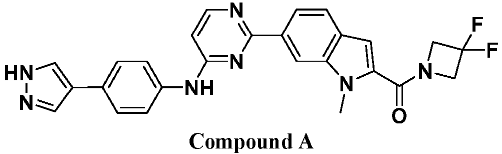 wherein the crystalline form I has an XRPD pattern comprising characteristic peaks at diffraction angles (20) of 8.3±0.2°, 12.6±0.2° and 18.5±0.2°.

2. The crystalline form I according to claim 1, wherein the crystalline form I has an XRPD pattern comprising characteristic peaks at diffraction angles (20) of 8.3±0.2°, 12.6±0.2°, 16.5±0.2°, 17.3±0.2°, 17.8±0.2°, 18.5±0.2°, 24.4±0.2°, 26.0±0.2° and 29.1±0.2°.

3. The crystalline form I according to claim 1 or 2, wherein the crystalline form I has an XRPD pattern comprising characteristic peaks at diffraction angles (20) of 8.3±0.2°, 11.2±0.2°, 12.6±0.2°, 16.5±0.2°, 17.3±0.2°, 17.8±0.2°, 18.5±0.2°, 19.5±0.2°, 19.8±0.2°, 20.6±0.2°, 21.0±0.2°, 21.7±0.2°, 23.7±0.2°, 24.4±0.2°, 25.5±0.2°, 26.0±0.2°, 29.1±0.2° and 33.6±0.2°.

4. The crystalline form I according to any one of claims 1-3, wherein the crystalline form I has a DSC graph comprising endothermic peaks at 105°C, 146°C and 245°C.

5. The crystalline form I according to any one of claims 1-4, wherein in a thermogravimetric analysis, the crystalline form I has a weight loss of 3.4% when heated to 169°C.

6. A pharmaceutical composition comprising the crystalline form I according to any one of claims 1-5, and one or more pharmaceutically acceptable carriers.

7. The crystalline form I according to any one of claims 1-5 for use in the prevention or treatment of a disease mediated by the Rho-associated protein kinase, wherein the disease is selected from:
a cardiovascular disorder, wherein the cardiovascular disorder is hypertension, atherosclerosis, restenosis, cardiac hypertrophy, cerebral ischemia, cerebral vasospasm, or erectile dysfunction;
inflammation, wherein the inflammation is asthma, cardiovascular inflammation, ulcerative colitis, or renal inflammation;
a central nervous system disorder, wherein the central nervous system disorder is neuronal degeneration or spinal cord injury; and the central nervous system disorder is preferably Huntington's disease, Parkinson's disease, Alzheimer's disease, Amyotrophic lateral sclerosis, or multiple sclerosis;
an arterial thrombotic disorder, wherein the arterial thrombotic disorder is platelet aggregation, or leukocyte aggregation;
a neoplastic disease, wherein the neoplastic disease is a lymphoma, carcinoma, leukemia, astrocytoma, soft tissue sarcoma, sarcoma, or blastoma, and the carcinoma is squamous cell cancer, small-cell lung cancer, pituitary cancer, esophageal cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, bladder cancer, liver cancer, breast cancer, colon cancer, colorectal cancer, endometrial or uterine carcinoma, salivary gland carcinoma, kidney cancer, prostate cancer, vulval cancer, thyroid cancer, brain cancer, endometrial cancer, testis cancer, cholangiocarcinoma, gallbladder carcinoma, gastric cancer, melanoma, or head and neck cancer;
a metabolic syndrome;
insulin resistance;
hyperinsulinemia;
type 2 diabetes;
glucose intolerance;
osteoporosis; and
an ocular disorder, wherein the ocular disorder is ocular hypertension, age related macular degeneration, choroidal neovascularization, diabetic macular edema, iris neovascularization, uveitis, glaucoma, or retinitis of prematurity, and the glaucoma is primary open-angle glaucoma, acute angle-closure glaucoma, pigmentary glaucoma, congenital glaucoma, normal tension glaucoma, secondary glaucoma or neo vascular glaucoma.

8. The crystalline form I according to any one of claims 1-5 for use in the prevention or treatment of an autoimmune disorder, wherein the autoimmune disorder is selected from rheumatoid arthritis, systemic lupus erythematosus, psoriasis, Crohn's disease, atopic dermatitis, eczema, and graft-versus-host disease.

9. The crystalline form I according to any one of claims 1-5 for use in the prevention or treatment of a fibrotic disorder, wherein the fibrotic disorder is selected from liver fibrosis, lung fibrosis, and kidney fibrosis.

10. The crystalline form I according to any one of claims 1-5 for use in the prevention or treatment of a disease mediated by the Rho-associated protein kinase, wherein the disease is selected from lupus nephritis, atherosclerosis, rheumatoid arthritis, hemangioma, angiofibroma, lung fibrosis, psoriasis, corneal graft rejection, insulin-dependent diabetes mellitus, multiple sclerosis, myasthenia gravis, Chron's disease, autoimmune nephritis, primary biliary cirrhosis, acute pancreatitis, allograph rejection, allergic inflammation, contact dermatitis, delayed hypersensitivity, inflammatory bowel disease, septic shock, osteoporosis, osteoarthritis, neuronal inflammation, Osier-Weber syndrome, restenosis, fungal infection, parasitic infection, and viral infection.

11. A method for preparing the crystalline form I of compound A monohydrochloride monohydrate according to any one of claims 1-5, comprising
adding a ketone solvent to compound A, wherein the ketone solvent preferably is a ketone having 3-6 carbon atoms, more preferably, acetone, butanone, methyl ethyl ketone, methyl isobutyl ketone and diethyl ketone,
stirring under heating, preferably heating to 40-80°C, more preferably, 50°C or 60°C,
and then adding water, wherein the volume ratio of the ketone solvent to water is preferably (1-15):1, preferably 10:1, so as to fully dissolve compound A;
then adding hydrochloric acid, wherein the concentration of hydrochloric acid is preferably 2-15 mol/L, preferably 12 mol/L, and the molar ratio of compound A to HCl is 1:(1-1.3),
stirring under heating, preferably heating to 40-80°C, more preferably, 50°C or 60°C,
filtering and optionally drying to obtain the crystalline form.

## Patentansprüche

1. Kristalline Form I von Verbindung A-Monohydrochlorid-Monohydrat, 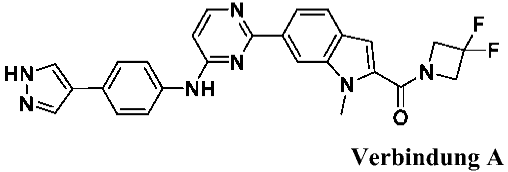 wobei die kristalline Form I ein XRPD-Muster aufweist, das charakteristische Spitzen bei Beugungswinkeln (2θ) von 8,3±0,2°, 12,6±0,2° und 18,5±0,2° umfasst.

2. Kristalline Form I nach Anspruch 1, wobei die kristalline Form I ein XRPD-Muster aufweist, das charakteristische Spitzen bei Beugungswinkeln (2θ) von 8,3±0,2°, 12,6±0,2°, 16,5±0,2°, 17,3±0,2°, 17,8±0,2°, 18,5±0,2°, 24,4±0,2°, 26,0±0,2° und 29,1±0,2° umfasst.

3. Kristalline Form I nach Anspruch 1 oder 2, wobei die kristalline Form I ein XRPD-Muster aufweist, das charakteristische Spitzen bei Beugungswinkeln (2θ) von 8,3±0,2°, 11,2±0,2°, 12,6±0,2°, 16,5±0,2°, 17,3±0,2°, 17,8±0,2°, 18,5±0,2°, 19,5±0,2°, 19,8±0,2°, 20,6±0,2°, 21,0±0,2°, 21,7±0,2°, 23,7±0,2°, 24,4±0,2°, 25,5±0,2°, 26,0±0,2°, 29,1±0,2° und 33,6±0,2° umfasst.

4. Kristalline Form I nach einem der Ansprüche 1-3, wobei die kristalline Form I einen DSC-Graphen aufweist, der endotherme Spitzen bei 105 °C, 146 °C und 245 °C umfasst.

5. Kristalline Form I nach einem der Ansprüche 1-4, wobei die kristalline Form I in einer thermogravimetrischen Analyse einen Gewichtsverlust von 3,4 % aufweist, wenn sie auf 169 °C erhitzt wird.

6. Pharmazeutische Zusammensetzung, umfassend die kristalline Form I nach einem der Ansprüche 1-5 und einen oder mehrere pharmazeutisch unbedenkliche Träger.

7. Kristalline Form I nach einem der Ansprüche 1-5 zur Verwendung bei der Vorbeugung oder Behandlung einer Krankheit, die durch die Rho-assoziierte Proteinkinase vermittelt wird, wobei die Krankheit ausgewählt ist aus Folgenden:
einer kardiovaskulären Erkrankung, wobei die kardiovaskuläre Erkrankung Bluthochdruck, Atherosklerose, Restenose, Herzhypertrophie, zerebrale Ischämie, zerebraler Vasospasmus oder erektile Dysfunktion ist;
Entzündung, wobei die Entzündung Asthma, kardiovaskuläre Entzündung, Colitis ulcerosa oder Nierenentzündung ist;
einer Erkrankung des zentralen Nervensystems, wobei die Erkrankung des zentralen Nervensystems eine neuronale Degeneration oder eine Rückenmarksverletzung ist; und die Erkrankung des zentralen Nervensystems bevorzugt Huntington-Krankheit, Parkinson-Krankheit, Alzheimer-Krankheit, amyotrophe Lateralsklerose oder Multiple Sklerose ist;
einer arteriellen thrombotischen Erkrankung, wobei die arterielle thrombotische Erkrankung eine Thrombozytenaggregation oder Leukozytenaggregation ist;
einer neoplastischen Krankheit, wobei die neoplastische Krankheit ein Lymphom, Karzinom, Leukämie, Astrozytom, Weichteilsarkom, Sarkom oder Blastom ist und das Karzinom Plattenepithelkrebs, kleinzelliger Lungenkrebs, Hypophysenkrebs, Speiseröhrenkrebs, nichtkleinzelliger Lungenkrebs, Adenokarzinom der Lunge, Plattenepithelkarzinom der Lunge, Peritonealkrebs, Leberzellkarzinom, Magen-Darm-Krebs, Bauchspeicheldrüsenkrebs, Glioblastom, Gebärmutterhalskrebs, Eierstockkrebs, Blasenkrebs, Leberkrebs, Brustkrebs, Kolonkrebs, Kolorektalkrebs, Endometrium- oder Gebärmutterkarzinom, Speicheldrüsenkarzinom, Nierenkrebs, Prostatakrebs, Vulvakrebs, Schilddrüsenkrebs, Hirnkrebs, Endometriumkrebs, Hodenkrebs, Cholangiokarzinom, Gallenbasenkarzinom, Magenkrebs, Melanom oder Kopf- und Halskrebs ist;
einem metabolischen Syndrom;
Insulinresistenz;
Hyperinsulinämie;
Typ-2-Diabetes;
Glukoseintoleranz;
Osteoporose; und
einer Augenerkrankung, wobei die Augenerkrankung okuläre Hypertension, altersbedingte Makuladegeneration, choroidale Neovaskularisation, diabetisches Makulaödem, Irisneovaskularisation, Uveitis, Glaukom oder Frühgeborenenretinitis ist und das Glaukom primäres Offenwinkelglaukom, akutes Winkelblockglaukom, Pigmentglaukom, kongenitales Glaukom, Normaldruckglaukom, sekundäres Glaukom oder neovaskuläres Glaukom ist.

8. Kristalline Form I nach einem der Ansprüche 1-5 zur Verwendung bei der Vorbeugung oder Behandlung einer Autoimmunerkrankung, wobei die Autoimmunerkrankung ausgewählt ist aus rheumatoider Arthritis, systemischem Lupus erythematodes, Psoriasis, Morbus Crohn, atopischer Dermatitis, Ekzem und Graft-versus-Host-Krankheit.

9. Kristalline Form I nach einem der Ansprüche 1-5 zur Verwendung bei der Vorbeugung oder Behandlung einer fibrotischen Erkrankung, wobei die fibrotische Erkrankung ausgewählt ist aus Leberfibrose, Lungenfibrose und Nierenfibrose.

10. Kristalline Form I nach einem der Ansprüche 1-5 zur Verwendung bei der Vorbeugung oder Behandlung einer Krankheit, die durch die Rho-assoziierte Proteinkinase vermittelt wird, wobei die Krankheit ausgewählt ist aus Lupusnephritis, Atherosklerose, rheumatoider Arthritis, Hämangiom, Angiofibrom, Lungenfibrose, Psoriasis, Hornhauttransplantatabstoßung, insulinabhängigem Diabetes mellitus, Multipler Sklerose, Myasthenia gravis, Morbus Crohn, Autoimmunnephritis, primärer biliärer Zirrhose, akuter Pankreatitis, Allographenabstoßung, allergischer Entzündung, Kontaktdermatitis, verzögerter Überempfindlichkeit, entzündlicher Darmerkrankung, septischem Schock, Osteoporose, Osteoarthritis, neuronaler Entzündung, Osier-Weber-Syndrom, Restenose, Pilzinfektion, parasitärer Infektion und Virusinfektion.

11. Verfahren zur Herstellung der kristallinen Form I von Verbindung-A-Monohydrochlorid-Monohydrat nach einem der Ansprüche 1-5, umfassend:
Hinzufügen eines Ketonlösungsmittels zu Verbindung A, wobei das Ketonlösungsmittel bevorzugt ein Keton mit 3-6 Kohlenstoffatomen ist, besonders bevorzugt Aceton, Butanon, Methylethylketon, Methylisobutylketon und Diethylketon,
Rühren unter Erwärmen, bevorzugt Erwärmen auf 40-80 °C, besonders bevorzugt 50 °C oder 60 °C,
und dann Hinzufügen von Wasser, wobei das Volumenverhältnis des Ketonlösungsmittels zu Wasser bevorzugt (1-15):1 ist, bevorzugt 10:1, um Verbindung A vollständig aufzulösen;
dann Hinzufügen von Salzsäure, wobei die Konzentration von Salzsäure bevorzugt 2-15 mol/l, bevorzugt 12 mol/l ist und das Molverhältnis von Verbindung A zu HCl 1:(1-1,3) ist,
Rühren unter Erwärmen, bevorzugt Erwärmen auf 40-80 °C, besonders bevorzugt 50 °C oder 60 °C,
Filtern und optional Trocknen, um die kristalline Form zu erlangen.

## Revendications

1. Forme cristalline I du monochlorhydrate monohydraté du composé A, 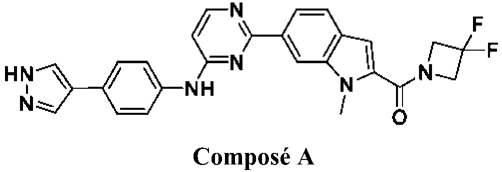 dans laquelle la forme cristalline I présente un diagramme XRPD comprenant des pics caractéristiques à des angles de diffraction (2θ) de 8,3±0,2°, 12,6±0,2° et 18,5±0,2°.

2. Forme cristalline I selon la revendication 1, dans laquelle la forme cristalline I présente un diagramme XRPD comprenant des pics caractéristiques à des angles de diffraction (2θ) de 8,3±0,2°, 12,6±0,2°, 16,5±0,2°, 17,3±0,2°, 17,8±0,2°, 18,5±0,2°, 24,4±0,2°, 26,0±0,2° et 29,1±0,2°.

3. Forme cristalline I selon la revendication 1 ou 2, dans laquelle la forme cristalline I présente un diagramme XRPD comprenant des pics caractéristiques à des angles de diffraction (2θ) de 8,3±0,2°, 11,2±0,2°, 12,6±0,2°, 16,5±0,2°, 17,3±0,2°, 17,8±0,2°, 18,5±0,2°, 19,5±0,2°, 19,8±0,2°, 20,6±0,2°, 21,0±0,2°, 21,7±0,2°, 23,7±0,2°, 24,4±0,2°, 25,5±0,2°, 26,0±0,2°, 29,1±0,2° et 33,6±0,2°.

4. Forme cristalline I selon l'une quelconque des revendications 1 à 3, dans laquelle la forme cristalline I présente un graphique DSC comprenant des pics endothermiques à 105 °C, 146 °C et 245 °C.

5. Forme cristalline I selon l'une quelconque des revendications 1 à 4, dans laquelle, lors d'une analyse thermogravimétrique, la forme cristalline I présente une perte de masse de 3,4 % lorsqu'elle est chauffée à 169 °C.

6. Composition pharmaceutique comprenant la forme cristalline I selon l'une quelconque des revendications 1 à 5 et un ou plusieurs supports acceptables pharmaceutiquement.

7. Forme cristalline I selon l'une quelconque des revendications 1 à 5, destinée à être utilisée dans la prévention ou le traitement d'une maladie médiée par la protéine kinase associée à Rho, dans laquelle la maladie est choisie parmi :
un trouble cardiovasculaire, dans laquelle le trouble cardiovasculaire est l'hypertension, l'athérosclérose, la resténose, l'hypertrophie cardiaque, l'ischémie cérébrale, le vasospasme cérébral ou la dysfonction érectile ;
une inflammation, dans laquelle l'inflammation est l'asthme, une inflammation cardiovasculaire, la colite ulcéreuse ou une inflammation rénale ;
un trouble du système nerveux central, dans laquelle le trouble du système nerveux central est une dégénérescence neuronale ou une lésion de la moelle épinière ; et le trouble du système nerveux central est de préférence la maladie de Huntington, la maladie de Parkinson, la maladie d'Alzheimer, la sclérose latérale amyotrophique ou la sclérose en plaques ;
un trouble thrombotique artériel, dans laquelle le trouble thrombotique artériel est l'agrégation plaquettaire ou l'agrégation leucocytaire ;
une maladie néoplasique, dans laquelle la maladie néoplasique est un lymphome, un carcinome, une leucémie, un astrocytome, un sarcome des tissus mous, un sarcome ou un blastome, et le carcinome est un cancer épidermoïde, un cancer du poumon à petites cellules, un cancer de l'hypophyse, un cancer de l'œsophage, un cancer du poumon non à petites cellules, un adénocarcinome du poumon, un carcinome épidermoïde du poumon, un cancer du péritoine, un cancer hépatocellulaire, un cancer gastro-intestinal, un cancer du pancréas, un glioblastome, un cancer du col de l'utérus, un cancer de l'ovaire, un cancer de la vessie, un cancer du foie, un cancer du sein, un cancer du côlon, un cancer colorectal, un carcinome de l'endomètre ou de l'utérus, un carcinome des glandes salivaires, un cancer du rein, un cancer de la prostate, un cancer de la vulve, un cancer de la thyroïde, un cancer du cerveau, un cancer de l'endomètre, un cancer des testicules, un cholangiocarcinome, un carcinome de la vésicule biliaire, un cancer gastrique, un mélanome ou un cancer de la tête et du cou ;
un syndrome métabolique ;
la résistance à l'insuline ;
l'hyperinsulinémie ;
le diabète de type 2 ;
l'intolérance au glucose ;
l'ostéoporose ; et
un trouble oculaire, dans laquelle le trouble oculaire est l'hypertension oculaire, la dégénérescence maculaire liée à l'âge, la néovascularisation choroïdienne, l'œdème maculaire diabétique, la néovascularisation de l'iris, l'uvéite, le glaucome ou la rétinite du prématuré, et le glaucome est un glaucome primitif à angle ouvert, un glaucome aigu à angle fermé, un glaucome pigmentaire, un glaucome congénital, un glaucome à tension normale, un glaucome secondaire ou un glaucome néovasculaire.

8. Forme cristalline I selon l'une quelconque des revendications 1 à 5, destinée à être utilisée dans la prévention ou le traitement d'un trouble auto-immun, dans laquelle le trouble auto-immun est choisi parmi la polyarthrite rhumatoïde, le lupus érythémateux disséminé, le psoriasis, la maladie de Crohn, la dermatite atopique, l'eczéma et la maladie du greffon contre l'hôte.

9. Forme cristalline I selon l'une quelconque des revendications 1 à 5, destinée à être utilisée dans la prévention ou le traitement d'un trouble fibrotique, dans laquelle le trouble fibrotique est choisi parmi la fibrose hépatique, la fibrose pulmonaire et la fibrose rénale.

10. Forme cristalline I selon l'une quelconque des revendications 1 à 5, destinée à être utilisée dans la prévention ou le traitement d'une maladie médiée par la protéine kinase associée à Rho, dans laquelle la maladie est choisie parmi la néphrite lupique, l'athérosclérose, la polyarthrite rhumatoïde, l'hémangiome, l'angiofibrome, la fibrose pulmonaire, le psoriasis, le rejet de greffe de cornée, le diabète sucré insulino-dépendant, la sclérose en plaques, la myasthénie grave, la maladie de Crohn, la néphrite auto-immune, la cirrhose biliaire primaire, la pancréatite aiguë, le rejet d'allogreffe, une inflammation allergique, la dermatite de contact, l'hypersensibilité retardée, les maladies inflammatoires chroniques de l'intestin, le choc septique, l'ostéoporose, l'ostéoarthrite, une inflammation neuronale, le syndrome d'Osier-Weber, la resténose, une infection fongique, une infection parasitaire et une infection virale.

11. Procédé permettant la préparation de la forme cristalline I du monochlorhydrate monohydraté du composé A selon l'une quelconque des revendications 1 à 5, comprenant
l'ajout d'un solvant cétonique au composé A, dans lequel le solvant cétonique est de préférence une cétone comportant 3 à 6 atomes de carbone, idéalement, l'acétone, la butanone, la méthyléthylcétone, la méthylisobutylcétone et la diéthylcétone,
l'agitation sous chauffage, de préférence chauffage à 40 à 80 °C, idéalement à 50 °C ou 60 °C,
puis l'ajout d'eau, dans lequel le rapport volumique du solvant cétonique à l'eau est de préférence de (1-15):1, de préférence de 10:1, de manière à dissoudre complètement le composé A ;
puis l'ajout d'acide chlorhydrique, dans lequel la concentration d'acide chlorhydrique est de préférence de 2 à 15 mol/l, de préférence de 12 mol/l, et le rapport molaire du composé A à l'HCl est de 1:(1-1,3),
l'agitation sous chauffage, de préférence chauffage à 40 à 80 °C, idéalement à 50 °C ou 60 °C,
la filtration et, éventuellement, le séchage pour obtenir la forme cristalline.
